# EUROPÄISCHE PATENTSCHRIFT

(11) **EP 0 351 790 B1**
(45) Veröffentlichungstag und Bekanntmachung des Hinweises auf die Patenterteilung: **01.06.1994**
(21) Anmeldenummer: 89113171.6
(22) Anmeldetag: 18.07.1989
(51) Int. Cl.: G01N 33/66, G01N 33/96

(54) **Verfahren zur Bestimmung von Fructosamin**
Method for the determination of fructosamine
Méthode pour la détermination de fructosamine

(30) Priorität: 19.07.1988 DE 3824562
(43) Veröffentlichungstag der Anmeldung: 24.01.1990
(73) Patentinhaber: BOEHRINGER MANNHEIM GMBH, 68298 Mannheim (DE)
(72) Erfinder: Vogt, Bernd, Dr. med., D-8132 Tutzing (DE); Lessmann, Hans-Dieter, Dr. rer. nat., D-6945 Hirschber-Grosssachsen (DE); Klein, Christian, Dr. rer. nat., D-8120 Weilheim (DE); Treiber, Wolfgang, D-8124 Seeshaupt (DE)
(74) Vertreter: Huber, Bernhard, Dipl.-Chem.

(56) Entgegenhaltungen:
- CLIN. CHEM., Band 31, Nr. 9, 1985, Seiten 1550-1554; J.R. BAKER et al.: "Use ofprotein-based standards in automated colorimetric determinations offructosamine in serum"
- CLINICAL CHEMISTRY, Band 33, Nr. 3, 1987, Seiten 447-448; M. LEVER et al.:"More on serum fructosamine assay"
- CLINICA CHEMICA ACTA, Band 127, Nr. 1, 1982, Seiten 87-95; R.N. JOHNSON et al.:"Fructosamine: a new approach to the estimation of serum glycosylprotein. An
- index of diabetic control"
- CLINICAL CHEMISTRY, Band 36, Nr. 1, 1990, Seiten 136-139; E.D. SCHLEICHER etal.: "Standardization of serum fructosamine assays"

## Beschreibung

Die Erfindung betrifft ein Verfahren zur Bestimmung von Fructosamin in Körperflüssigkeiten sowie eine hierzu geeignete Standardlösung.

Bei diabetischer Stoffwechsellage werden durch die im Blut vorhandene überschüssige Glucose Proteine glucosyliert. Dabei reagiert die Carbonylgruppe der Glucose zuerst mit freien Proteinaminoresten unter Bildung von Schiff'schen Basen. Durch Amadori-Umlagerung entstehen dann Fructosamine, die eine stabile Ketoaminbindung aufweisen. Wegen der Stabilität dieser Ketoaminbindung ist die Halbwertszeit der Fructosamine praktisch identisch mit der der Serumproteine. Fructosamine sind daher als sogenannte integrale Diabetesparameter geeignet, d.h. sie erlauben eine Aussage über den mittleren Blutglucosespiegel der letzten Wochen. Als ein Indikator wurde dazu bisher Glucosylhämoglobin, das als HbA₁ bezeichnet wird, bestimmt. Die Überwachung dieses Parameters ist für eine Langfristkontrolle des Zuckerstoffwechsels geeignet. Da das Glucosylhämoglobin aufgrund seiner langen Halbwertszeit nur längerfristige Änderungen der Stoffwechsellage dokumentiert und die Trägheit des Hämoglobinabbaus dazu führt, daß kurzfristige Stoffwechselschwankungen nicht erkennbar werden, ist dieser Parameter nicht ausreichend für eine mittelfristige Kontrolle der Stoffwechselführung. Andererseits kann der Zuckerstoffwechsel bei Diabetikern durch Bestimmung des Blutglucosespiegels überwacht werden. Da jedoch der Blutglucosespiegel starken Schwankungen unterliegt, gibt er dem Arzt nur Auskunft über die zum Zeitpunkt der Blutentnahme vorhandene Stoffwechsellage. Diese Lücke zwischen Kurzfristüberwachung durch Blutserumspiegel und Langfristkontrolle durch Bestimmung von HbA_{1c} füllt nun die Bestimmung der als Fructosamine bezeichneten glycosylierten Proteine. Verschiedene Studien haben gezeigt, daß die Serumfructosaminbestimmung eine zuverlässige spezifische und praktikable Methode zur Überwachung von Diabetikern ist.

Bekannte Verfahren zur Bestimmung des Fructosamins, wie z. B. in Johnson et al., Clin. Chem. Acta (1982) 127, 87-95 beschrieben, beruhen darauf, daß das Fructosamin, das in wäßrigem alkalischem Milieu in Enolform vorliegt und in dieser Form leicht oxidiert werden kann, umgesetzt wird mit einem Oxidationsmittel, das in reduzierter Form farbig ist, beispielsweise Tetrazoliumsalz. Der dabei gebildete Formazanfarbstoff kann dann photometrisch gemessen werden und ist der Menge an Fructosamin proportional. Ein weiteres Verfahren zur Bestimmung von Fructosamin, welches auf einer HPLC-Trennung beruht, ist in J. Clin. Chem. Clin. Biochem. 19 (1981) 81-87 beschrieben.

Um nun eine genaue Bestimmung zu ermöglichen, ist es notwendig, mit Standardlösungen Eichkurven aufzustellen, um dann bei Durchführung einer Bestimmung in einer Probelösung den erhaltenen Wert durch Vergleich mit der Eichkurve festzustellen. Weiterhin ist es zur Präzisionskontrolle von Bestimmungsmethoden und zum Eichen von Analysenautomaten notwendig, Standardlösungen mit bekanntem Gehalt zu verwenden. Standardlösungen, die für diese Zwecke eingesetzt werden, müssen den zu bestimmenden Meßparameter in bekannter Konzentration enthalten. Die Konzentration des Parameters muß im medizinisch relevanten Meßbereich liegen. Die Handhabung der Standardlösungen muß einfach sein und insbesondere müssen sie eine möglichst lange Haltbarkeit besitzen.

Die bisher bekannten Standardlösungen für die Fructosaminbestimmung erfüllen einige oder mehrere dieser Voraussetzungen nicht. So werden einerseits Kontroll- bzw. Eichseren verwendet, die die Serumfructosamin-Konzentration in Form von Modellsubstanzen nachbilden. Üblicherweise wird hierzu 1-Deoxy-1-morpholino-fructose (DMF) verwendet. Derartige Primärstandards werden beispielsweise durch Einwiegen von bestimmten Mengen DMF in Albuminlösungen hergestellt. Nachteil dieser bekannten Standardlösungen ist es, daß die Modellsubstanz DMF eine andere Struktur hat, sich daher ganz anders verhält und eine andere Reaktivität besitzt, als die Serumfructosamine, so daß ein Vergleich erhaltener Werte mit einer mit DMF hergestellten Eichkurve viel zu hohe Werte liefert. Man drückt daher auch die so erhaltenen Werte als DMF- Einheiten aus.

Anhand dieser Primärstandards können dann Standardlösungen, die Serumfructosamine enthalten, geeicht werden (Siehe z.B. Clin. Chem. (1985) 31, 1550-1554). Derartige (Sekundär-)Standards zeigen jedoch nach mehrtägiger Lagerung bei +35°C eine starke Instabilität der Serumfructosamin-Konzentration. Bei gleichzeitiger Anwesenheit von Glucose wird eine Zunahme des Fructosamingehalts von 200 % und mehr beobachtet, die auf eine weitergehende nichtenzymatische Proteinglucosylierung zurückzuführen ist. Bei Glucoseausschluß beobachtet man umgekehrt eine Abnahme des Fructosaminwerts.

Es war daher Aufgabe der vorliegenden Erfindung, sowohl Primär- als auch Sekundär-Standardlösungen für die Serumfructosamin-Bestimmung bereitzustellen, die einfach zu handhaben sind, deren Konzentration leicht zu bestimmen ist und die über lange Zeit gelagert werden können, ohne ihre Stabilität zu verlieren.

Diese Aufgabe wird gelöst durch ein Verfahren zur Bestimmung von Fructosamin in Körperflüssigkeiten, welches dadurch gekennzeichnet ist, daß man zur Calibrierung als Standardlösung eine Lösung verwendet, die ein Peptid oder Protein, dessen Aminosäureeinheiten zu mindestens 25% aus Lysin und/oder Ornithin bestehen und das in glykosilierter Form vorliegt, enthält.

Unter Fructosamin im Sinne der Erfindung sind nichtenzymatisch glycosylierte Serumproteine (glycierte Serumproteine) wie z.B. glycosyliertes Albumin, Immunglobulin oder Fibrinogen sowie nichtenzymatisch glycosylierte Blutproteine wie z.B. Lysin-glycosyliertes Hämoglobin und glycosyliertes Erythrozytenmembranprotein zu verstehen.

Erfindungsgemäß wird eine Standardlösung zur Verfügung gestellt, die den Parameter Serumfructosamin in glycosyliertem Serumprotein ähnlicher Form enthält und deren Serumfructosaminwert einfach durch C/N-Analyse feststellbar ist. Ein weiterer Vorteil der erfindungsgemäßen Standardlösung ist, daß diese als vollsynthetische Matrix eingesetzt werden kann, von der keinerlei Infektionsgefahr ausgeht.

Dazu wird eine Lösung artefiziell glycosylierter Peptide oder Proteine hergestellt und die jeweils gewünschte Konzentration eingestellt. Durch Variation der zugesetzten Mengen können so Standardlösungen für normale und pathologische Serumfructosamin-Konzentrationen bereitgestellt werden. Die Herstellung des glycosylierten Peptids oder Proteins kann beispielsweise analog einem in J.F. Day et al., J. Biol. Chem. 254 Nr. 3 (1979) 595-597 beschriebenen Verfahren erfolgen. Dabei wird eine wäßrige Serumalbuminlösung mit Glucose bei 25°C 8 Tage inkubiert und anschließend zur Entfernung freier Glucose dialysiert.

Bei dem erfindungsgemäßen Verfahren wird als Standardlösung eine Lösung verwendet, die ein Peptid oder Protein, dessen Aminosäureeinheiten zu mindestens 25% aus Lysin und/oder Ornithin bestehen und das in glykosilierter Form vorliegt, enthält. Das eingesetzte Peptid oder Protein sollte mindestens 5 Lysin- bzw. Ornithineinheiten, gegebenenfalls mit weiteren Aminosäureeinheiten, aufweisen. In einer bevorzugten Ausführungsform wird ein Polylysin, ein Polyornithin oder ein Copolymerisat aus Lysin und Ornithin verwendet. Derartige Polyaminosäuren können beispielsweise nach Methods in Enzymology Band III, S. 540 (1957), Academic Press N.Y. und J. Biochem. 85 (1962) 233 hergestellt werden.

Bevorzugt wird ein Peptid verwendet, dessen Molekulargewicht größer ist als etwa 1000. Die Größe des Peptids oder Proteins ist an sich nicht kritisch. Allerdings sollte das Molekül noch löslich sein, so daß Proteine mit Molekulargewichten bis ca. 300.000, bevorzugt 150.000 verwendet werden können.

Das verwendete Peptid oder Protein wird in an sich bekannter Weise glykosiliert durch Inkubation des Peptids oder Proteins mit Glucose. Bevorzugt wird die glykosilierung des Peptids oder Proteins so lange durchgeführt, bis 5 bis 35% aller Aminoseitengruppen glykosiliert sind. Anschließend wird das Reaktionsprodukt ausgiebig dialysiert.

Als Peptid oder Protein können sowohl natürliche als auch synthetisch hergestellte verwendet werden. Besonders bevorzugt wird Poly-L-Lysin oder Poly-L-Ornithin in glykosilierter Form verwendet, wobei der Fructosamingehalt durch einfache C/N-Elementaranalyse bestimmt werden kann.

Das Peptid oder Protein wird in wäßrigem Medium gelöst. Ebenso kann auch als Basislösung Humanserum verwendet werden.

Es ist vorteilhaft, wenn die Lösung glucosefrei ist, da auf diese Weise eine lange Lagerstabilität gewährleistet werden kann. Bei der Verwendung von Humanserum muß dieses daher glucosefrei gemacht werden. Dies kann beispielsweise geschehen, indem das Humanserum gegen einen glucosefreien Puffer dialysiert wird.

Die Standardlösung kann weiterhin für Eichseren übliche Substanzen enthalten. So können beispielsweise Aufhellungsmittel, Stabilisierungsmittel, Detergentien und Konservierungsstoffe zugesetzt werden. Als Aufhellungsmittel kann beispielsweise Pentaerythrit verwendet werden. Als Stabilisierungsmittel sind insbesondere Zink und EDTA geeignet. Als Konservierungsstoffe können z. B. Phenole oder Antibiotika eingesetzt werden. Auch andere dem Fachmann bekannte Hilfsstoffe können verwendet werden.

Die Herstellung der erfindungsgemäß verwendeten Standardlösung erfolgt durch Vermischen der einzelnen Bestandteile und gegebenenfalls Einstellen des pH's durch Zugabe des Puffersystems.

Üblicherweise wird die Standardlösung anschließend keimfrei filtriert und kann dann für eine längere Lagerung lyophilisiert werden.

In einer besonders bevorzugten Ausführungsform wird der erfindungsgemäße Standard in saurer Lösung, vorzugsweise bei einem pH-Wert unter 6, besonders bevorzugt zwischen 0 und 4 aufbewahrt. Überraschenderweise wird der Standard dadurch so gut stabilisiert, daß eine Lyophilisation nicht erforderlich ist und der Standard über lange Zeit in Lösung aufbewahrt werden kann. Der erforderliche pH-Wert kann durch Zugabe von anorganischen oder organischen Säuren wie z.B. Salzsäure, Zitronensäure oder Essigsäure oder auch durch Pufferzugabe erreicht werden.

Die erfindungsgemäß verwendete Standardlösung ist äußerst stabil und liefert steile Eichkurven, so daß eine genaue und empfindliche Bestimmung der Fructosamine möglich wird. Ein Vergleich von mit dem erfindungsgemäßen Verfahren erhaltenen Werten für die Fructosaminkonzentration mit Werten, die nach anderen bekannten Verfahren, die zur Urkalibration verwendet werden, erhalten wurden zeigt, daß das erfindungsgemäße Verfahren sehr einfach durchzuführen ist und mit der mittels ¹⁴C-Glucose-Einbau ermittelten Urkalibration gut übereinstimmt.

Gegenstand der Erfindung ist weiterhin eine Standardlösung zur Bestimmung von Fructosamin in Körperflüssigkeiten, die dadurch gekennzeichnet ist, daß sie ein Peptid oder Protein, dessen Aminosäureeinheiten zu mindestens 25% aus Lysin und/oder Ornithin bestehen und das in glykosylierter Form vorliegt, enthält.

Die erfindungsgemäß zur Verfügung gestellte Standardlösung hat die gleiche Struktur und damit Reaktivität wie natürlich vorkommendes Fructosamin. Daher eignet sich die erfindungsgemäße Standardlösung zur Urstandardisierung von Sekundärstandards und Kontrollseren ebenso wie als Standardlösung zur Kalibrierung von Fructosaminbestimmungen.

Die Erfindung wird durch die folgenden Beispiele erläutert.

### Beispiel 1

### Herstellung von Polylysin-Fructosamin

In einem 250 ml Rundkolben wurden 500 mg Poly-L-Lysin (Hersteller: Sigma Chemie, BRD; mittl. MG 39000) und 1200 mg D(+)-Glucose eingewogen und mit 50 ml Eisessig suspendiert. Man rührte eine Stunde bei Raumtemperatur, wodurch man eine homogene Suspension erhielt. Danach wurde langsam 50 ml Pyridin zugetropft, die Suspension 8 Tage bei Raumtemperatur gerührt und anschließend in 300 ml entsalztes Wasser gegossen. Dabei wurde eine klare Lösung erhalten, die auf 10 ml konzentriert wurde. Mit 300 ml 0,02%igem HCl wurde dann auf ca. 350 ml aufgefüllt und wiederum auf 10 ml konzentriert. Dieser Vorgang wurde sechsmal mit 0,02%iger HCl wiederholt, bis der Pyridingeruch verschwunden war. Anschließend wurde noch sechsmal mit entsalztem Wasser gewaschen, bis das Filtrat neutral war. Aus einem Volumen von 50 ml wäßriger Lösung wurde das Produkt lyophilisiert.

Der Glycosylierungsgrad des erhaltenen Produkts wird durch Elementaranalyse über das C/N-Verhältnis bestimmt. Für eine Lysineinheit beträgt das C/N-Verhältnis 3,0, für eine glycosylierte Lysineinheit 6,0.

Im vorliegenden Beispiel liegt der Glycosylierungsgrad bei 30% aller Lysinseitengruppen.

### Beispiel 2

Es wurden verschiedene Kalibrationslösungen in Kontrollseren auf Humanserumbasis in einem Fructosamintest kalibriert. Das Reagenz hatte die folgende Zusammensetzung: 2,2% nichtionisches Detergens, 4 U/ml Uricase, 0,5 mmol/l Nitrotetrazoliumblau (NBT) in 0,2 M Carbonatpuffer, pH 10,30.

Jeweils 50 µl Probe Humanserum mit unterschiedlichem Fructosamingehalt wurden mit jeweils 1000 µl Reagenz gemischt und die Extinktionszunahme wurde am Photometer zwischen der 10. und der 15. Minute bei 546 nm und 37°C gegen einen Reagenzienleerwert gemessen.

Zur Kalibrierung wurden die folgenden Lösungen verwendet:
a) eine Polylysinfructosaminlösung, deren Fructosamingehalt zuvor mittels C/N-Elementaranalyse bestimmt wurde;
b) eine Polylysinfructosaminlösung, deren Fructosamingehalt nach Inkubation mit 14 C Glucose und nachfolgender gründlicher Dialyse in einem Szintillationszähler bestimmt wurde;
c) eine Humanserumalbuminlösung, deren Fructosamingehalt durch in vitro Glykosylierung mit 14 C Glucose nach Johnson et al., Clin. Chem. 32, 368-370 (1986) bestimmt wurde.

Das Ergebnis für diese verschiedenen Standardlösungen und Kontrollseren ist in Tabelle 1 zusammengefaßt. Die Ergebnisse zeigen, daß glykosyliertes Polylysin die gleiche Reaktivität im Farbtest hat, wie physiologisch vorkommendes Fructosamin.

**Tabelle 1**

| | Methode a | Methode b | Methode c |
|---|---|---|---|
| Probe 1 | 268 | 267 | 268 |
| Probe 2 | 279 | 269 | 274 |
| Probe 3 | 515 | 491 | 512 |
| Probe 4 | 524 | 504 | 517 |
| Probe 5 | 390 | 387 | 390 |
| Probe 6 | 398 | 391 | 395 |

### Beispiel 3

Es wurde die Stabilität erfindungsgemäßer Standardlösungen nach Zusatz von Säuren in verschiedenen Konzentrationen untersucht. Dazu wurden Lösungen von gemäß Beispiel 1 hergestelltem Polylysinfructosamin in der entsprechenden Säure gelöst. Eine Testreihe unverdünnter Lösungen wurden drei Wochen bei -18°C und eine weitere Testreihe jeweils drei Wochen bei 35°C gelagert. Anschließend wurde die Fructosaminwiederfindung bestimmt. Die Ergebnisse sind der Tabelle 2 zu entnehmen. Es zeigt sich, daß auch nach dreiwöchiger Lagerung unter Temperaturbelastung der nachweisbare Fructosamingehalt sich praktisch nicht verändert hat.

**Tabelle 2**

| Säure | Fructosaminwiederfindung nach | |
|---|---|---|
| | 3 Wochen/-18°C | 3 Wochen/35°C |
| 0,01 mol/l HCl | 102,3 % | 101,1 % |
| 0,1 mol/l HCl | 100,4 % | 100,1 % |
| 0,01 mol/l Essigsäure | 100,6 % | 98,0 % |
| 0,1 mol/l Essigsäure | 100,4 % | 97,9 % |
| 0,1 mol/l Citronensäure | 99,1 % | 97,1 % |
| 0,01 mol/l Citronensäure | 99,9 % | 96,6 % |
| 0,001 mol/l Citronensäure | 99,8 % | 94,9 % |

### Beispiel 4

### Herstellung von Poly-L-Lysin-L-Phenylalanin-Fructosamin

Analog Beispiel 1 setzt man Poly-L-Lysin-L-Phenylalanin (Firma Sigma Chemie, mittleres Molekulargewicht 46000) mit Glucose um. Mit Hilfe der Elementaranalyse erhält man aus dem C/N-Verhältnis einen Glycosylierungsgrad von 38,5%.

### Beispiel 5

### Herstellung von Poly-L-Ornithin-Fructosamin

Analog Beispiel 1 setzt man Poly-L-Ornthin (Firma Sigma Chemie, mittleres Molekulargewicht 32000) mit Glucose um.

### Beispiel 6

### Herstellung von Poly-L-Ornithin-L-Leucin-Fructosamin

Analog Beispiel 1 setzt man Poly-L-Ornithin-L-Leucin, 1:1 (Firma Sigma Chemie, mittleres Molekulargewicht 35000) mit Glucose um.

### Beispiel 7

### Herstellung von Poly-L-Lysin-Fructosamin verschiedener Beladungsgrade.

Analog Beispiel 1 setzt man Poly-L-Lysin (Firma Sigma Chemie, mittleres Molekulargewicht 39000) mit Glucose um.

Bei einer Reaktionszeit von 15 Stunden bei Raumtemperatur erhält man ein Produkt, das lt. C/N-Verhältnis aus der Elementaranalyse zu 5,1% glycosyliert ist.
Bei einer Reaktionszeit von 64 Stunden bei Raumtemperatur erhält man ein Produkt, das lt. C/N-Verhältnis aus der Elementaranalyse zu 8,1% glycosyliert ist.
Bei einer Reaktionszeit von 120 Stunden bei Raumtemperatur erhält man ein Produkt, das lt. C/N-Verhältnis aus der Elementaranalyse zu 14,8% glycosyliert ist.

### Beispiel 8

### Herstellung von Poly-L-Lysin-Fructosamin mit niedrigem Molekulargewicht

Analog Beispiel 1 setzt man Poly-L-Lysin (Firma Sigma Chemie, mittleres Molekulargewicht 3300) mit Glucose um.

Nach dem C/N-Verhältnis aus der Elementaranalyse ist das Produkt zu 49% glycosyliert.

### Beispiel 9

### Herstellung von Poly-L-Lysin-Fructosamin mit höherem Molekulargewicht

Analog Beispiel 1 setzt man Poly-L-Lysin (Firma Sigma Chemie, mittleres Molekulargewicht 102000) mit Glucose um.

Nach dem C/N-Verhältnis aus der Elementaranalyse ist das Produkt zu 28% glycosyliert.

## Patentansprüche

1. Verfahren zur Bestimmung von Fructosamin in Körperflüssigkeiten,
**dadurch gekennzeichnet,**
daß man zur Calibrierung als Standardlösung eine Lösung verwendet, die ein Peptid oder Protein, dessen Aminosäureeinheiten zu mindestens 25% aus Lysin und/oder Ornithin bestehen und das in glycosylierter Form vorliegt, enthält.

2. Verfahren nach Anspruch 1,
**dadurch gekennzeichnet,**
daß ein Peptid oder Protein verwendet wird, bei dem mindestens 80% der darin enthaltenen Aminosäureeinheiten aus Lysin und/oder Ornithin bestehen.

3. Verfahren nach einem der vorhergehenden Ansprüche,
**dadurch gekennzeichnet,**
daß man ein Peptid oder Protein verwendet, bei dem 5 bis 50% aller Aminoseitengruppen glycosyliert sind.

4. Verfahren nach einem der vorhergehenden Ansprüche,
**dadurch gekennzeichnet,**
daß der pH-Wert der Lösung kleiner als 6 ist.

5. Verfahren nach einem der vorhergehenden Ansprüche,
**dadurch gekennzeichnet,**
daß man als Basislösung für die Standardlösung Humanserum verwendet.

6. Standardlösung zur Bestimmung von Fructosamin in Körperflüssigkeiten,
**dadurch gekennzeichnet,**
daß sie ein Peptid oder Protein, dessen Aminosäureeinheiten zu mindestens 25% aus Lysin und/oder Ornithin bestehen und das in glycosylierter Form vorliegt, enthält.

7. Standardlösung nach Anspruch 6,
**dadurch gekennzeichnet,**
daß sie ein Peptid oder Protein enthält, bei dem mindestens 80% der darin enthaltenen Aminosäureeinheiten aus Lysin und/oder Ornithin bestehen.

8. Standardlösung nach einem der Ansprüche 6 oder 7,
**dadurch gekennzeichnet,**
daß sie ein Peptid oder Protein enthält, bei dem 5 bis 50% aller Aminoseitengruppen glycosyliert sind.

9. Standardlösung nach einem der vorhergehenden Ansprüche,
**dadurch gekennzeichnet,**
daß ihr pH-Wert kleiner als 6 ist.

10. Verwendung der Standardlösung nach einem der Ansprüche 6 bis 9 zur Eichung eines Standards zur Bestimmung von Fructosamin in Körperflüssigkeiten.

## Claims

1. Process for the determination of fructosamine in body fluids, characterised in that, for the calibration, as standard solution one uses a solution which contains a peptide or protein, the amino acid units of which consist of at least 25% lysine and/or ornithine and is present in glycosylated form.

2. Process according to claim 1, characterised in that a peptide or protein is used in which at least 80% of the amino acid units contained therein consist of lysine and/or ornithine.

3. Process according to one of the preceding claims, characterised in that one uses a peptide or protein in which 5 to 50% of all amino side groups are glycosylated.

4. Process according to one of the preceding claims, characterised in that the pH value of the solution is less than 6.

5. Process according to one of the preceding claims, characterised in that one uses human serum as base solution for the standard solution.

6. Standard solution for the determination of fructosamine, characterised in that it contains a peptide or protein, the amino acid units of which consist of at least 25% lysine and/or ornithine and is present in glycosylated form.

7. Standard solution according to claim 6, characterised in that it contains a peptide or protein in which at least 80% of the amino acid units contained therein consist of lysine and/or ornithine.

8. Standard solution according to one of claims 6 or 7, characterised in that it contains a peptide or protein in which 5 to 50% of all amino side groups are glycosylated.

9. Standard solution according to one of claims 6 to 8, characterised in that its pH value is less then 6.

10. Use of the standard solution according to one of claims 6 to 9 for the calibration of a standard for the determination of fructosamine in body fluids.

## Revendications

1. Procédé de détermination de la fructosamine dans les fluides corporels, caractérisé en ce que l'on utilise comme solution standard pour l'étalonnage une solution qui contient un peptide ou une protéine dont les unités d'acides aminés consistent à raison d'au moins 25% en lysine et/ou en ornithine et qui est sous foie glycosylée.

2. Procédé selon la revendication 1, caractérisé en ce que l'on utilise un peptide ou une protéine dans lequel ou laquelle au moins 80% des unités d'acides aminés qu'il ou elle contient consistent en lysine et/ou en ornithine.

3. Procédé selon l'une des revendications précédentes, caractérisé en ce que l'on utilise un peptide ou une protéine dans lequel ou laquelle 5 à 50% de tous les groupes latéraux aminés sont glycosylés.

4. Procédé selon l'une des revendications précédentes, caractérisé en ce que le pH de la solution est inférieur à 6.

5. Procédé selon l'une des revendications précédentes, caractérisé en ce que l'on utilise comme solution de base pour la solution standard du sérum humain.

6. Solution standard pour la détermination de la fructosamine dans les fluides corporels, caractérisée en ce qu'elle contient un peptide ou une protéine dont les unités d'acides aminés consistent à raison d'au moins 25% en lysine et/ou en ornithine et qui est sous forme glycosylée.

7. Solution standard selon la revendication 6, caractérisée en ce qu'elle contient un peptide ou une protéine dans lequel ou laquelle au moins 80% des unités d'acides aminés qu'il ou elle contient consistent en lysine et/ou en ornithine.

8. Solution standard selon l'une des revendications 6 ou 7, caractérisée en ce qu'elle contient un peptide ou une protéine dans lequel ou laquelle 5 à 50% de tous les groupes latéraux aminés sont glycosylés.

9. Solution standard selon l'une des revendications précédentes, caractérisée en ce que son pH est inférieur à 6.

10. Utilisation de la solution standard selon l'une des revendications 6 à 9 pour l'étalonnage d'un standard pour la détermination de la fructosamine dans les fluides corporels.
